# EUROPEAN PATENT APPLICATION

(11) **EP 4 311 364 A1**
(43) Date of publication of application: **24.01.2024**
(21) Application number: 22770566.2
(22) Date of filing: 16.03.2022
(51) Int. Cl.: H04W 76/10

(54) **METHOD FOR TRANSMITTING BRAIN ELECTRICAL SIGNAL, AND DEVICE**

(30) Priority: 19.03.2021 CN 202110296316
(71) Applicant: Sceneray Co., Ltd., Jiangsu 215123 (CN)
(72) Inventor: CHEN, Lei, Suzhou, Jiangsu 215123 (CN); ZHU, Weiran, Suzhou, Jiangsu 215123 (CN); CHEN, Jinghua, Suzhou, Jiangsu 215123 (CN); CHANG, Yueyan, Suzhou, Jiangsu 215123 (CN)
(74) Representative: Cabinet Beaumont
(86) International application number: PCT/CN2022/081213
(87) International publication number: WO 2022/194213

(57) **Abstract**

Provided are a method for transmitting a brain electrical signal, and a device. The method for transmitting a brain electrical signal is applied to a brain electrical signal acquisition device associated with a patient and includes the following: establishing a two-way communication with a charging device; receiving a charging instruction sent by the charging device, and interacting with the charging device according to the charging instruction to perform charging; and in a case where the brain electrical signal acquisition device is charging, receiving a brain electrical related data fetch instruction sent by the charging device, and transmitting brain electrical related data of the patient acquired by the brain electrical signal acquisition device to the charging device according to the brain electrical related data fetch instruction.

## Description

This application claims priority to Chinese Patent Application No. 202110296316.X filed with the China National Intellectual Property Administration (CNIPA) on Mar. 19, 2021, the disclosure of which is incorporated herein by reference in its entirety.

### TECHNICAL FIELD

The present application relates to the technical field of medical devices, for example, a method for transmitting a brain electrical signal, and a device.

### BACKGROUND

An implantable nerve stimulation system, including a nerve stimulator and a stimulation electrode implanted in the deep brain, is configured to treat nervous system disorders such as Parkinson's disease and epilepsy, and mental diseases such as addiction and obsessive-compulsive disorder. Clinically, an implantable nerve stimulator of Medtronic's Percept model can continuously acquire and record brain electrical characteristic signals of a patient for a long time after operation and may be used for long-term monitoring of disease.

A manner of acquiring electrical signals in the deep brain for a long time after operation is mainly as follows: in a non-program control state, that is, during daily stimulation of the patient, brain electrical signals are acquired continuously, and an abnormally fluctuating signal is recorded and stored in the memory of the nerve stimulator. The limitation of the nerve stimulator is that a large-capacity memory chip cannot be placed inside the nerve stimulator due to the volume of the nerve stimulator. Therefore, the storage space of the nerve stimulator is limited, and the nerve stimulator cannot continuously and periodically monitor brain electrical signals of a patient and cannot take a measure timely when an abnormality occurs.

### SUMMARY

The present application provides a method for transmitting a brain electrical signal, and a device, so as to overcome a defect in the related art that a nerve stimulator cannot continuously and periodically acquire brain electrical signals of a patient.

The present application provides a method for transmitting a brain electrical signal, which is applied to a brain electrical signal acquisition device associated with a patient. The method includes the steps described below.

A two-way communication with a charging device is established.

A charging instruction sent by the charging device is received, and the brain electrical signal acquisition device interacts with the charging device according to the charging instruction to perform charging.

In the case where the brain electrical signal acquisition device is charging, a brain electrical related data fetch instruction sent by the charging device is received, and brain electrical related data of the patient acquired by the brain electrical signal acquisition device is transmitted to the charging device according to the brain electrical related data fetch instruction.

In an embodiment of the present application, the step in which the brain electrical signal acquisition device interacts with the charging device according to the charging instruction to perform the charging includes the step described below.

The brain electrical signal acquisition device interacts with the charging device according to the charging instruction to adjust a charging parameter, and the charging is performed according to the charging parameter, where the charging parameter is used for enabling a charging rate of the brain electrical signal acquisition device to meet a set requirement.

In an embodiment of the present application, the step in which the brain electrical related data of the patient acquired by the brain electrical signal acquisition device is transmitted to the charging device according to the brain electrical related data fetch instruction includes the steps described below.

Brain electrical related data stored in the brain electrical signal acquisition device is transmitted to the charging device according to the brain electrical related data fetch instruction, where the brain electrical related data is obtained after the brain electrical signal acquisition device converts and stores an acquired brain electrical related signal of the patient before the brain electrical signal acquisition device is charged.

Alternatively, in the case where the brain electrical signal acquisition device is charging, a brain electrical related signal of the patient is acquired, the brain electrical related signal is converted into brain electrical related data, and the brain electrical related data is transmitted to the charging device.

In an embodiment of the present application, the step in which the brain electrical signal acquisition device converts and stores the acquired brain electrical related signal of the patent includes the step described below.

The brain electrical related signal of the patient is acquired according to a set sampling parameter, the brain electrical related signal is converted into the brain electrical related data, and the brain electrical related data is compressed and stored in a memory of the brain electrical signal acquisition device, where the sampling parameter includes sampling duration, a sampling rate, a sampling channel, sampling data and a compression ratio.

The present application provides a method for transmitting a brain electrical signal, which is applied to a charging device. The method includes the steps described below.

A charging instruction is sent to a brain electrical signal acquisition device, and the charging device interacts with the brain electrical signal acquisition device to charge the brain electrical signal acquisition device.

A brain electrical related data fetch instruction is generated and sent to the brain electrical signal acquisition device in a charging state, and brain electrical related data sent by the brain electrical signal acquisition device is received.

The brain electrical related data is transmitted to a server.

In an embodiment of the present application, the step in which the brain electrical related data is transmitted to the server includes the steps described below.

A communication connection with the server is established, and whether the communication connection is normal is determined.

In response to the communication connection being normal, the received brain electrical related data is transmitted to the server.

In response to the communication connection being abnormal, the received brain electrical related data is stored in the charging device, where the storage capacity of the charging device is at least 10 times the storage capacity of the brain electrical signal acquisition device.

The present application provides a method for transmitting a brain electrical signal, which is applied to a wireless communication system including a brain electrical signal acquisition device and a charging device. The method includes the steps described below.

A two-way communication between the brain electrical signal acquisition device associated with a patient and the charging device is established.

The charging device sends a charging signal to the brain electrical signal acquisition device.

The brain electrical signal acquisition device receives a charging instruction sent by the charging device and interacts with the charging device according to the charging instruction to perform charging.

The charging device generates and sends a brain electrical related data fetch instruction to the brain electrical signal acquisition device in a charging state.

In the case where the brain electrical signal acquisition device is charging, the brain electrical related data fetch instruction sent by the charging device is received, and brain electrical related data of the patient acquired by the brain electrical signal acquisition device is transmitted to the charging device according to the brain electrical related data fetch instruction.

The charging device transmits the brain electrical related data to a server.

The present application provides a brain electrical signal acquisition device, which is associated with a patient. The device includes a communication module, a charging instruction receiving module and a transmission module.

The communication module is configured to establish a two-way communication with a charging device.

The charging instruction receiving module is configured to receive a charging instruction sent by the charging device and interact with the charging device according to the charging instruction to perform charging.

The transmission module is configured to, in the case where the brain electrical signal acquisition device is charging, receive a brain electrical related data fetch instruction sent by the charging device and transmit brain electrical related data of the patient acquired by the brain electrical signal acquisition device to the charging device according to the brain electrical related data fetch instruction.

The present application provides a charging device. The device includes a charging instruction sending module, a first transmission module and a second transmission module.

The charging instruction sending module is configured to send a charging signal to a brain electrical signal acquisition device and interact with the brain electrical signal acquisition device to charge the brain electrical signal acquisition device.

The first transmission module is configured to generate and send a brain electrical related data fetch instruction to the brain electrical signal acquisition device in a charging state and receive brain electrical related data sent by the brain electrical signal acquisition device.

The second transmission module is configured to transmit the brain electrical related data to a server.

### BRIEF DESCRIPTION OF DRAWINGS

FIG. 1 is a flowchart illustrating a two-way communication established between a brain electrical signal acquisition device and a charging device according to an embodiment of the present application.
FIG. 2 is a flowchart illustrating transmission of a brain electrical signal according to an embodiment of the present application.
FIG. 3 is a flowchart illustrating another two-way communication established between a brain electrical signal acquisition device and a charging device according to an embodiment of the present application.
FIG. 4 is a flowchart illustrating another two-way communication established between a brain electrical signal acquisition device and a charging device according to an embodiment of the present application.
FIG. 5 is a flowchart illustrating transmission of a brain electrical signal according to an embodiment of the present application.
FIG. 6 is a structural diagram of a brain electrical signal acquisition device according to an embodiment of the present application.
FIG. 7 is a structural diagram of a charging device according to an embodiment of the present application.
FIG. 8 is a structural diagram of another charging device according to an embodiment of the present application.

### Reference list

- 100: brain electrical signal acquisition device
- 101: communication module
- 102: acquisition and storage module
- 103: charging instruction receiving module
- 104: transmission module
- 105: stimulation module
- 200: charging device
- 201: charging instruction sending module
- 202: first transmission module
- 203: second storage module
- 204: second transmission module
- 300: server

### DETAILED DESCRIPTION

The present application is described hereinafter in detail in conjunction with drawings and embodiments so that those skilled in the art can understand and implement the present application.

The wireless communication system in the present application is widely arranged to provide multiple communication services such as charging. The wireless communication system includes a brain electrical signal acquisition device and a charging device. The brain electrical signal acquisition device in the present application is, for example, a rechargeable and implantable nerve stimulator. The rechargeable and implantable nerve stimulator needs to be charged regularly on time according to power consumption.

### Embodiment one

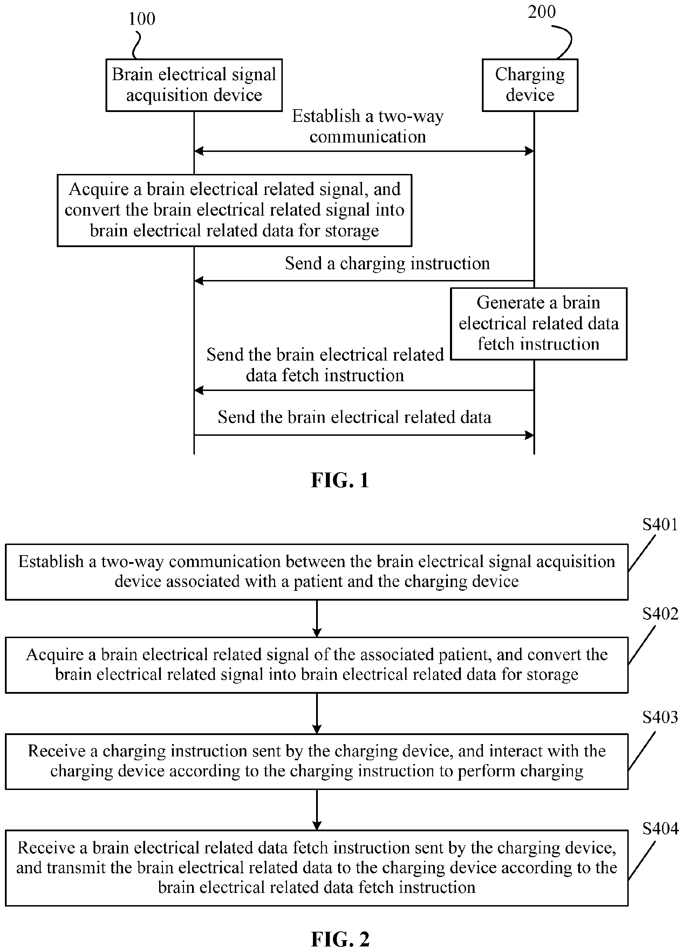

FIG. 1 is a flowchart illustrating that a two-way communication between a brain electrical signal acquisition device 100 and a charging device 200 is established according to an embodiment of the present application.

Referring to FIG. 1, the brain electrical signal acquisition device 100 associated with a patient may establish the two-way communication with the charging device 200, and the brain electrical signal acquisition device 100 may perform bi-directional information transmission with the charging device 200.

Transmission between the brain electrical signal acquisition device 100 associated with the patient and the charging device 200 is wireless, and at least two communication channels are provided between the brain electrical signal acquisition device 100 and the charging device 200. One of the two communication channels is used by the charging device 200 to charge the brain electrical signal acquisition device 100, and the other communication channel is used by the brain electrical signal acquisition device 100 to transmit brain electrical related data acquired and stored by the brain electrical signal acquisition device 100 to the charging device 200 so as to release the storage space of the brain electrical signal acquisition device 100.

The method for transmitting a brain electrical signal provided in this embodiment and applied to the brain electrical signal acquisition device 100 is described below.

FIG. 2 is a flowchart illustrating transmission of a brain electrical signal according to an embodiment of the present application.

Referring to FIG. 2, this embodiment provides a method for transmitting a brain electrical signal applied to the brain electrical signal acquisition device 100. The method includes the steps described below.

In S401, a two-way communication is established between the brain electrical signal acquisition device 100 associated with a patient and the charging device 200.

In S402, a brain electrical related signal of the associated patient is acquired, and the brain electrical related signal is converted into brain electrical related data for storage.

Exemplarily, the brain electrical related signal of the associated patient is acquired according to a set sampling parameter, the brain electrical related signal is converted into the brain electrical related data, and the brain electrical related data is compressed and stored in the memory of the brain electrical signal acquisition device 100. The sampling parameter includes the sampling duration, the sampling rate, the sampling channel, sampling data and the compression ratio. For example, the sampling duration per day is 24 hours, the sampling rate is 200 Hz, the sampling channel is 1, and the sampling data is 2 bytes. The brain electrical related data is compressed and stored at a lossless compression ratio of 1:2. The volume of the brain electrical related data is about 132 Mbit, and the data may be stored in an external random-access memory (RAM) (1 Gbit) of a nerve stimulator. If the volume of the brain electrical related data exceeds the storage space of the RAM, a cyclic covering storage manner may be used.

In S403, a charging instruction sent by the charging device 200 is received, and the brain electrical signal acquisition device 100 interacts with the charging device 200 according to the charging instruction to perform charging.

Exemplarily, the charging device 200 sends the charging instruction to the brain electrical signal acquisition device 100, the brain electrical signal acquisition device 100 interacts with the charging device 200 to adjust a charging parameter after receiving the charging instruction, and the charging is performed according to the charging parameter to enable the charging rate of the brain electrical signal acquisition device 100 to meet a set requirement.

In S404, a brain electrical related data fetch instruction sent by the charging device 200 is received, and the brain electrical related data is transmitted to the charging device 200 according to the brain electrical related data fetch instruction.

Exemplarily, the charging device 200 generates and sends the brain electrical related data fetch instruction to the brain electrical signal acquisition device 100 when charging the brain electrical signal acquisition device 100, and the brain electrical signal acquisition device 100 performs the transmission of the brain electrical related data after receiving the brain electrical related data fetch instruction. The brain electrical related data is wirelessly transmitted to the charging device 200.

In the present application, a communication manner of simultaneous bi-directional transmission is used. While the brain electrical signal acquisition device 100 is charging, the brain electrical related data stored in the brain electrical signal acquisition device 100 is transmitted to the charging device 200 to release the storage space of the brain electrical signal acquisition device 100 so that the brain electrical signal acquisition device 100 can continuously and periodically acquire brain electrical signals of a patient without adding an additional operation of the patient, thereby effectively overcoming the defect that a brain electrical signal acquisition device 100 in the related art cannot continuously and periodically acquire brain electrical signals of a patient due to the limited storage space, which has strong operability and is easy to promote.

### Embodiment two

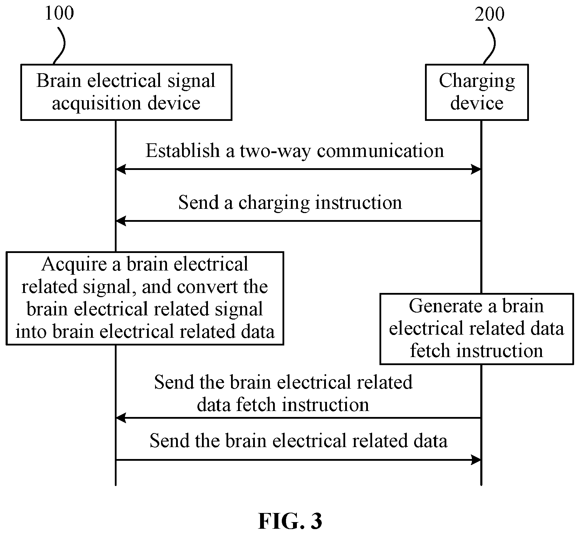

FIG. 3 is a flowchart illustrating another two-way communication established between a brain electrical signal acquisition device 100 and a charging device 200 according to an embodiment of the present application.

Referring to FIG. 3, the brain electrical signal acquisition device 100 associated with a patient may establish the two-way communication with the charging device 200.

The transmission between the brain electrical signal acquisition device 100 associated with the patient and the charging device 200 is wireless, and at least two communication channels are provided between the brain electrical signal acquisition device 100 and the charging device 200. One of the two communication channels is used by the charging device 200 to charge the brain electrical signal acquisition device 100, and the other communication channel is used by the brain electrical signal acquisition device 100 to transmit brain electrical related data, which is acquired by the brain electrical signal acquisition device 100 while the brain electrical signal acquisition device 100 is charging, to the charging device 200 in real time, so that the brain electrical related data acquired by the brain electrical signal acquisition device 100 does not occupy the storage space of the brain electrical signal acquisition device 100.

The method for transmitting a brain electrical signal provided in this embodiment and applied to the brain electrical signal acquisition device 100 is described below. Embodiment two is implemented based on the preceding embodiment one and expanded on the basis of embodiment one.

The difference between embodiment two and embodiment one is described below.

In embodiment two, while the brain electrical signal acquisition device 100 is charging, the brain electrical signal acquisition device 100 acquires a brain electrical related signal of the associated patient, converts the brain electrical related signal into brain electrical related data, and transmits the brain electrical related data to the charging device 200.

In this embodiment, the brain electrical signal acquisition device 100 acquires the brain electrical related data of the patient while charging and directly transmits the acquired brain electrical related data to the charging device 200 so that the brain electrical related data acquired by the brain electrical signal acquisition device 100 does not occupy the storage space of the brain electrical signal acquisition device 100. In this manner, operations of acquiring the brain electricity signal and transmitting the data are synchronously completed in a charging process of the brain electrical signal acquisition device 100, thereby omitting a subsequent operation of transmitting the brain electrical related data in the brain electrical signal acquisition device 100 to the charging device 200.

### Embodiment three

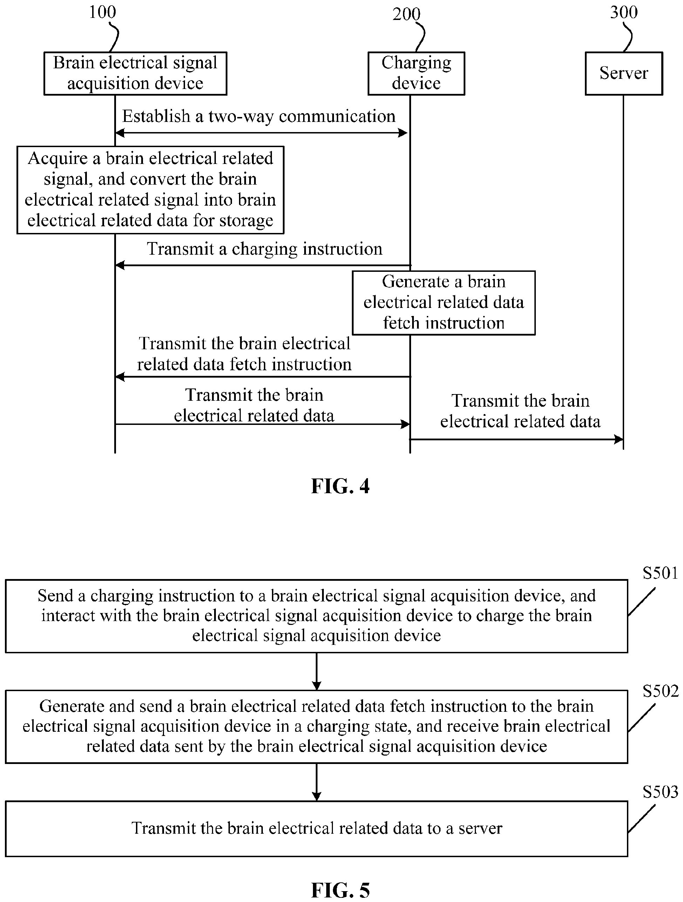

FIG. 4 is a flowchart illustrating another two-way communication established between a brain electrical signal acquisition device 100 and a charging device 200 according to an embodiment of the present application.

Referring to FIG. 4, the brain electrical signal acquisition device 100 associated with a patient may establish the two-way communication with the charging device 200, and the charging device 200 establishes a two-way communication with a server 300.

The brain electrical signal acquisition device 100 may perform bi-directional information transmission with the charging device 200, and the charging device 200 may perform bi-directional information transmission with the server 300. Transmission between the brain electrical signal acquisition device 100 associated with the patient and the charging device 200 and transmission between the charging device 200 and the server 300 may be wireless, and at least two communication channels are between the brain electrical signal acquisition device 100 and the charging device 200. One of the two communication channels is used by the charging device 200 to charge the brain electrical signal acquisition device 100, and the other communication channel is used by the brain electrical signal acquisition device 100 to transmit brain electrical related data acquired and stored by the brain electrical signal acquisition device 100 to the charging device 200 so as to release the storage space of the brain electrical signal acquisition device 100. In addition, one communication channel may be between the charging device 200 and the server 300, and the communication channel is used by the charging device 200 to transmit the brain electrical related data received by the charging device 200 to the server 300.

The method for transmitting a brain electrical signal provided in this embodiment and applied to the brain electrical signal acquisition device 100 is described below. Embodiment three is implemented based on the preceding embodiment one and expanded on the basis of embodiment one.

The difference between embodiment three and embodiment one is described below.

In embodiment three, the charging device 200 transmits the brain electrical related data to the server 300 after receiving the brain electrical related data. A communication connection is established between the charging device 200 and the server 300, and whether the communication connection is normal is determined. If a determination result is that the communication connection is normal, the brain electrical related data received by the charging device 200 is transmitted to the server 300, and if the determination result is that the communication connection is abnormal, the brain electrical related data received by the charging device 200 is stored in the charging device 200. The storage capacity of the charging device 200 is at least 10 times the storage capacity of the brain electrical signal acquisition device 100, and the storage of the charging device 200 is used for storing brain electrical related data which cannot be transmitted to the server 300 in time when the brain electrical signal acquisition device 100 is charging. Of course, for the brain electrical related data stored in the charging device 200, after the communication connection is normal, the charging device 200 transmits the brain electrical related data to the server 300 in real time. After receiving the brain electrical related data, the server 300 may decode the data according to a set compression ratio to facilitate the subsequent statistical analysis.

In the present application, the charging device 200 transmits the brain electrical related data to the server 300 after receiving the brain electrical related data, so as to release the storage space of the charging device 200, thereby receiving brain electrical related data sent by the brain electrical signal acquisition device 100 continuously for a long time without affecting the storage of other data in the charging device 200. Moreover, transmission of the brain electrical related data from the brain electrical signal acquisition device 100 to the server 300 is completed while the brain electrical signal acquisition device 100 is charging so that only the brain electrical related data in the server 300 needs to be processed subsequently without adding an additional operation of the patient, which is convenient to use.

### Embodiment four

FIG. 5 is a flowchart illustrating transmission of a brain electrical signal according to an embodiment of the present application.

Referring to FIG. 5, this embodiment provides a method for transmitting a brain electrical signal applied to a charging device 200. The method includes the steps described below.

In S501, a charging instruction is sent to a brain electrical signal acquisition device 100, and the charging device 200 interacts with the brain electrical signal acquisition device 100 to charge the brain electrical signal acquisition device 100.

The content that the charging device 200 charges the brain electrical signal acquisition device 100 has been described in detail in the preceding embodiments, which is not repeated here in this embodiment.

In S502, a brain electrical related data fetch instruction is generated and sent to the brain electrical signal acquisition device 100 in a charging state, and brain electrical related data sent by the brain electrical signal acquisition device 100 is received.

The content that the brain electrical signal acquisition device 100 transmits the brain electrical related data to the charging device 200 has been described in detail in the preceding embodiments, which is not repeated here in this embodiment.

In S503, the brain electrical related data is transmitted to a server 300.

The content that the brain electrical related data is transmitted to the server 300 has been described in detail in the preceding embodiments, which is not repeated here in this embodiment.

Moreover, for details of an effect of this embodiment, reference is made to the preceding embodiments, which is not repeated here in this embodiment.

### Embodiment five

This embodiment provides a method for transmitting a brain electrical signal applied to a wireless communication system. The wireless communication system includes a brain electrical signal acquisition device 100 and a charging device 200, where steps performed by the brain electrical signal acquisition device 100 are shown in FIG. 2, which are not repeated here in this embodiment, and steps performed by the charging device 200 are shown in FIG. 5, which are not repeated here in this embodiment.

### Embodiment six

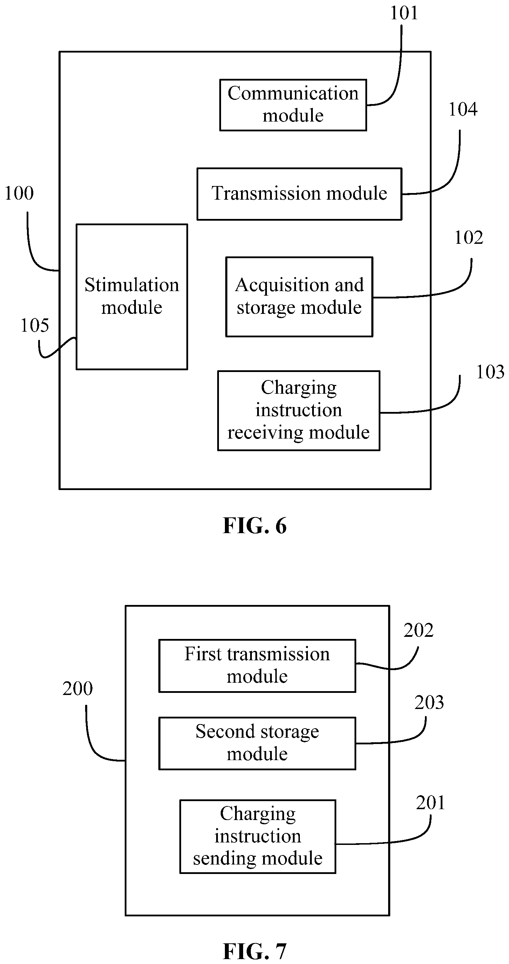

FIG. 6 is a structural diagram of a brain electrical signal acquisition device 100 according to an embodiment of the present application.

Referring to FIG. 6, the brain electrical signal acquisition device 100 provided in this embodiment includes a communication module 101, an acquisition and storage module 102, a charging instruction receiving module 103 and a transmission module 104.

The communication module 101 is configured to establish a two-way communication with a charging device 200, the acquisition and storage module 102 is configured to acquire a brain electrical related signal of an associated patient and convert the brain electrical related signal into brain electrical related data for storage, the charging instruction receiving module 103 is configured to receive a charging instruction sent by the charging device 200 and interact with the charging device 200 according to the charging instruction to perform charging, and the transmission module 104 is configured to, in a case where the brain electrical signal acquisition device 100 is charging, receive a brain electrical related data fetch instruction sent by the charging device 200 and transmit the brain electrical related data to the charging device 200 according to the brain electrical related data fetch instruction.

The acquisition and storage module 102 includes an amplifier module, an analog-to-digital conversion (ADC) module and a first storage module. The amplifier module is configured to acquire a weak brain electrical related signal, the ADC module is configured to convert the brain electrical related signal into brain electrical related data, and the first storage module is configured to store the brain electrical related data.

The charging instruction receiving module 103 includes a charging receiving circuit and a digital signal modulation circuit. The digital signal modulation circuit may be configured to transmit a charging-related parameter. The charging device 200 sends the charging instruction to the brain electrical signal acquisition device 100, and the brain electrical signal acquisition device 100 receives the charging instruction through the charging instruction receiving module 103, adjusts a charging parameter and interacts the charging parameter with the charging device 200 to enable the charging rate of the brain electrical signal acquisition device 100 to meet a set requirement when the brain electrical signal acquisition device 100 is charging according to the charging parameter.

The transmission module 104 may be a wireless module and is configured to transmit the brain electrical related data. The charging device 200 sends the brain electrical related data fetch instruction to the brain electrical signal acquisition device 100, and the brain electrical signal acquisition device 100 sends the brain electrical related data to the charging device 200 through the transmission module 104 after receiving the instruction. Of course, the transmission module 104 is further configured to bi-directionally transmit a program control instruction.

The brain electrical signal acquisition device 100 further includes a stimulation module 105, and the stimulation module 105 may receive a stimulation parameter set by a doctor and enables the brain electrical signal acquisition device 100 to output a set value.

### Embodiment seven

FIG. 7 is a structural diagram of a charging device 200 according to an embodiment of the present application.

Referring to FIG. 7, the charging device 200 in this embodiment includes a charging instruction sending module 201 and a first transmission module 202.

The charging instruction sending module 201 is configured to send a charging signal to a brain electrical signal acquisition device 100 and interact with the brain electrical signal acquisition device 100 to charge the brain electrical signal acquisition device 100, and the first transmission module 202 is configured to generate and send a brain electrical related data fetch instruction to the brain electrical signal acquisition device 100 in a charging state and receive brain electrical related data sent by the brain electrical signal acquisition device 100.

The charging instruction sending module 201 includes a charging sending circuit and a digital signal modulation circuit, and the digital signal modulation circuit may be configured to set and acquire a charging-related parameter. The charging device 200 charges the brain electrical signal acquisition device 100 through the charging instruction sending module 201, and the brain electrical signal acquisition device 100 receives a charging instruction through the charging instruction receiving module 103, adjusts a charging parameter and interacts the charging parameter with the charging device 200 to enable the charging rate to meet a set requirement in the case where the brain electrical signal acquisition device 100 is charging according to the charging parameter.

The first transmission module 202 may be a wireless module and is configured to transmit the brain electrical related data. The charging device 200 sends the brain electrical related data fetch instruction to the brain electrical signal acquisition device 100, and the brain electrical signal acquisition device 100 transmits the brain electrical related data to the first transmission module 202 of the charging device 200 through the transmission module 104 after receiving the instruction.

The charging device 200 further includes a second storage module 203. After the first transmission module 202 receives the brain electrical related data, the second storage module 203 is configured to store the brain electrical related data, where the storage capacity of the second storage module 203 included in the charging device 200 is at least 10 times the storage capacity of the first storage module included in the brain electrical signal acquisition device 100, thereby ensuring that the charging device 200 can continuously receive brain electrical related data from the brain electrical signal acquisition device 100.

### Embodiment eight

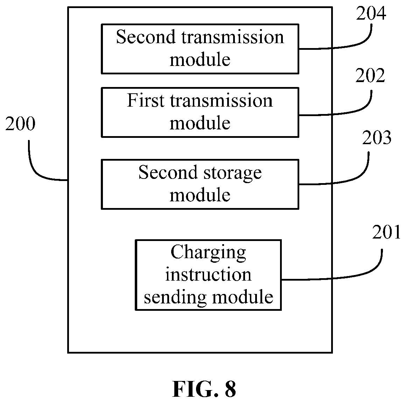

FIG. 8 is a structural diagram of another charging device 200 according to an embodiment of the present application.

The charging device 200 provided in this embodiment is described below. Embodiment eight is implemented based on the preceding embodiment seven and expanded on the basis of embodiment seven.

The difference between embodiment seven and embodiment eight is described below.

The charging device 200 in this embodiment includes a second transmission module 204 and is configured to transmit the brain electrical related data to a server 300. Therefore, at least unidirectional transmission exists between the first transmission module 202 and the second transmission module 204, that is, the first transmission module 202 transmits information to the second transmission module 204. Here, the first transmission module 202 and the second transmission module 204 may be wireless modules, such as Bluetooth modules or Wireless Fidelity (WiFi) modules or the 4th Generation mobile communication technology (4G) modules, or may be, of course, other modules, which is not limited in this embodiment. Therefore, while the brain electrical signal acquisition device 100 is charging, real-time transmission of the brain electrical related data between the first transmission module 202 and the second transmission module 204 can be completed without adding an additional operation, which has strong operability and is easy to promote.

### Embodiment nine

An embodiment of the present application provides a storage medium, which is configured as a computer-readable storage. The storage medium stores one or more programs, where the one or more programs are executable by one or more processors to implement the method for transmitting a brain electrical signal provided in the preceding embodiments.

The storage medium may include a computer storage medium (or a non-transitory medium) and a communication medium (or a transitory medium).

The embodiments of the present application may be provided as methods, systems or computer program products. Therefore, the present application may take the form of an entire hardware embodiment, an entire software embodiment or an embodiment combining both software and hardware aspects. Moreover, the present application may take the form of a computer program product implemented on one or more computer-usable storage media (including, but not limited to, a disk memory, a compact disc read-only memory (CD-ROM), an optical memory, and the like) that include computer-usable program codes.

The present application is described with reference to flowcharts and/or block diagrams of methods, devices (systems) and computer program products according to the embodiments of the present application. It is to be understood that each flow and/or block in the flowcharts and/or block diagrams and a combination of flows and/or blocks in the flowcharts and/or block diagrams are implemented by computer program instructions. These computer program instructions can be provided to a general-purpose computer, a special-purpose computer, an embedded processor or a processor of another programmable data processing device to produce a machine, so that instructions executed by a computer or a processor of another programmable data processing device produce an apparatus for implementing the functions specified in one or more flows in the flowcharts and/or one or more blocks in the block diagrams.

These computer program instructions may also be stored in a computer-readable memory which may direct the computer or other programmable data processing device to operate in a particular manner so that the instructions stored in the computer-readable memory produce a manufactured product including an instruction means. The instruction means implements the functions specified in one or more flows in the flowcharts and/or one or more blocks in the block diagrams.

These computer program instructions may also be loaded onto the computer or other programmable data processing device so that a series of operation steps are performed on the computer or other programmable device to produce processing implemented by the computer. Therefore, instructions executed on the computer or other programmable device provide steps for implementing the functions specified in one or more flows in the flowcharts and/or one or more blocks in the block diagrams.

## Claims

1. A method for transmitting a brain electrical signal, being applied to a brain electrical signal acquisition device associated with a patient and comprising:
establishing a two-way communication with a charging device;
receiving a charging instruction sent by the charging device, and interacting with the charging device according to the charging instruction to perform charging; and
in a case where the brain electrical signal acquisition device is charging, receiving a brain electrical related data fetch instruction sent by the charging device, and transmitting, according to the brain electrical related data fetch instruction, brain electrical related data of the patient acquired by the brain electrical signal acquisition device to the charging device.

2. The method according to claim 1, wherein interacting with the charging device according to the charging instruction to perform the charging comprises:
interacting with the charging device according to the charging instruction to adjust a charging parameter, and performing the charging according to the charging parameter, wherein the charging parameter is used for enabling a charging rate of the brain electrical signal acquisition device to meet a set requirement.

3. The method according to claim 1, wherein transmitting, according to the brain electrical related data fetch instruction, the brain electrical related data of the patient acquired by the brain electrical signal acquisition device to the charging device comprises:
transmitting, according to the brain electrical related data fetch instruction, brain electrical related data stored in the brain electrical signal acquisition device to the charging device, wherein the brain electrical related data is data obtained after the brain electrical signal acquisition device converts and stores an acquired brain electrical related signal of the patient before the brain electrical signal acquisition device is charged; or
in the case where the brain electrical signal acquisition device is charging, acquiring a brain electrical related signal of the patient, converting the brain electrical related signal into brain electrical related data, and transmitting the brain electrical related data to the charging device.

4. The method according to claim 3, wherein converting and storing, by the brain electrical signal acquisition device, the acquired brain electrical related signal of the patient comprises:
acquiring the brain electrical related signal of the patient according to a set sampling parameter, converting the brain electrical related signal into the brain electrical related data, and compressing and storing the brain electrical related data in a memory of the brain electrical signal acquisition device, wherein the sampling parameter comprises sampling duration, a sampling rate, a sampling channel, sampling data and a compression ratio.

5. A method for transmitting a brain electrical signal, being applied to a charging device and comprising:
sending a charging instruction to a brain electrical signal acquisition device, and interacting with the brain electrical signal acquisition device to charge the brain electrical signal acquisition device;
generating a brain electrical related data fetch instruction, sending the brain electrical related data fetch instruction to the brain electrical signal acquisition device in a charging state, and receiving brain electrical related data sent by the brain electrical signal acquisition device; and
transmitting the brain electrical related data to a server.

6. The method according to claim 5, wherein transmitting the brain electrical related data to the server comprises:
establishing a communication connection with the server, and determining whether the communication connection is normal;
in response to the communication connection being normal, transmitting the received brain electrical related data to the server; and
in response to the communication connection being abnormal, storing the received brain electrical related data in the charging device, wherein a storage capacity of the charging device is at least 10 times a storage capacity of the brain electrical signal acquisition device.

7. A method for transmitting a brain electrical signal, being applied to a wireless communication system, wherein the wireless communication system comprises a brain electrical signal acquisition device and a charging device; wherein the method comprises:
establishing a two-way communication between the brain electrical signal acquisition device associated with a patient and the charging device;
sending, by the charging device, a charging signal to the brain electrical signal acquisition device;
receiving, by the brain electrical signal acquisition device, a charging instruction sent by the charging device, and interacting with the charging device according to the charging instruction to perform charging;
generating, by the charging device, a brain electrical related data fetch instruction and sending the brain electrical related data fetch instruction to the brain electrical signal acquisition device in a charging state;
in a case where the brain electrical signal acquisition device is charging, receiving the brain electrical related data fetch instruction sent by the charging device, and transmitting brain electrical related data of the patient acquired by the brain electrical signal acquisition device to the charging device according to the brain electrical related data fetch instruction; and
transmitting, by the charging device, the brain electrical related data to a server.

8. A brain electrical signal acquisition device, being associated with a patient and comprising:
a communication module configured to establish a two-way communication with a charging device;
a charging instruction receiving module configured to receive a charging instruction sent by the charging device and interact with the charging device according to the charging instruction to perform charging; and
a transmission module configured to, in a case where the brain electrical signal acquisition device is charging, receive a brain electrical related data fetch instruction sent by the charging device and transmit brain electrical related data of the patient acquired by the brain electrical signal acquisition device to the charging device according to the brain electrical related data fetch instruction.

9. A charging device, comprising:
a charging instruction sending module configured to send a charging signal to a brain electrical signal acquisition device and interact with the brain electrical signal acquisition device to charge the brain electrical signal acquisition device;
a first transmission module configured to generate a brain electrical related data fetch instruction, send the brain electrical related data fetch instruction to the brain electrical signal acquisition device in a charging state, and receive brain electrical related data sent by the brain electrical signal acquisition device; and
a second transmission module configured to transmit the brain electrical related data to a server.

10. A storage medium, being configured as a computer-readable storage and storing at least one program, wherein the at least one program is executable by at least one processor to implement the method for transmitting a brain electrical signal according to any one of claims 1 to 7.
